# EUROPÄISCHE PATENTSCHRIFT

(11) **EP 3 624 879 B1**
(45) Veröffentlichungstag und Bekanntmachung des Hinweises auf die Patenterteilung: **09.09.2026**
(21) Anmeldenummer: 18721366.5
(22) Anmeldetag: 27.04.2018
(51) Int. Cl.: A61J 1/06, A61M 5/19, A61M 5/24, A61M 5/31, A61J 1/14, A61J 1/16

(54) **MEDIKAMENTENBEHÄLTER MIT EINEM ENDSTOPFEN, VERWENDUNG EINES STOPFENSICHERUNGSTEILS ZUM SICHERN EINES ENDSTOPFENS IN EINEM MEDIKAMENTENBEHÄLTER UND STOPFENSICHERUNGSTEIL**
MEDICATION CONTAINER HAVING AN END PLUG, USE OF A PLUG-SECURING PART TO SECURE AN END PLUG IN A MEDICATION CONTAINER AND PLUG-SECURING PART
RÉCIPIENT POUR MÉDICAMENTS MUNI D'UN BOUCHON DE FERMETURE, UTILISATION D'UNE PIÈCE DE FIXATION DE BOUCHON POUR FIXER UN BOUCHON DE FERMETURE DANS UN RÉCIPIENT POUR MÉDICAMENTS ET PIÈCE DE FIXATION DE BOUCHON

(30) Priorität: 16.05.2017 DE 102017208255
(43) Veröffentlichungstag der Anmeldung: 25.03.2020
(73) Patentinhaber: Vetter Pharma-Fertigung GmbH & Co. KG, 88212 Ravensburg (DE)
(72) Erfinder: GLOCKER, Joachim, 88214 Ravensburg (DE)
(74) Vertreter: Kordel, Mattias
(86) Internationale Anmeldenummer: PCT/EP2018/060972
(87) Internationale Veröffentlichungsnummer: WO 2018/210556

(56) Entgegenhaltungen:
- US-A1- 2012 078 171
- US-A1- 2012 118 139
- US-A1- 2013 110 039
- US-A1- 2014 339 112
- US-A1- 2016 184 522

## Beschreibung

Die Erfindung betrifft einen Medikamentenbehälter mit einem Endstopfen, eine Verwendung eines Stopfensicherungsteils zum Sichern eines Endstopfens in einem Medikamentenbehälter und ein Stopfensicherungsteil.

Stopfensicherungsteile, Medikamentenbehälter mit Endstopfen und die Verwendung des Stopfensicherungsteils zum Sichern des Endstopfens in dem Medikamentenbehälter sind bekannt (US 2012/118139 A1; US 2016/184522 A1; US 2012/078171 A1; US 2014/339112 A1; US 2013/110039 A1). So wird der Stopfen klassischerweise in Spritzen durch eine Fingerauflage mit integriertem Stopfensicherungsteil gesichert. Auch für Doppelkammerspritzen ist eine derartige Sicherung mittels der Fingerauflage bekannt. Karpulen, insbesondere Doppelkammerkarpulen haben jedoch üblicherweise keine Fingerauflage. Daher wird der Stopfen in Karpulen klassischerweise dadurch gesichert, dass die Karpule in einer Applikationsvorrichtung, insbesondere in einem Einwegpen montiert wird, sodass eine Kolbenstange der Applikationsvorrichtung den Endstopfen sichert.

Außerdem sind Transportboxen zur Aufnahme mehrerer Karpulen bekannt, um den Endstopfen insbesondere während eines Lufttransports zu sichern und an einer Position zu halten. Dazu sind im Boden der Transportbox Erhöhungen vorgesehen, welche in den Grundkörper des Medikamentenbehälters eindringen, um einen darin angeordneten Endstopfen zu sichern. Der Medikamentenbehälter in einer solchen Transportbox ist also klassischerweise zwischen dem Boden und dem Deckel der Transportbox angeordnet, während die vom Boden ausgehende Erhöhung in den Innenraum des Grundkörpers des Medikamentenbehälters eindringt, um während des Transports eine Verlagerung des dort angeordneten Endstopfens in Richtung des Bodens zu begrenzen.

Nachteilig an diesem bekannten Stand der Technik ist, dass eine Sicherung des Endstopfens mittels einer Fingerauflage nur dann möglich ist, wenn der Medikamentenbehälter eine solche Fingerauflage aufweist. Die Endstopfensicherung durch Montage der Karpule in einer Applikationsvorrichtung ist dagegen umständlich und teuer. Die Sicherung des Endstopfens mittels einer Transportbox hat den Nachteil, dass die Erhöhung im Boden der Box genau auf die Position des Endstopfens - entlang einer Mittelachse des Endstopfens - in dem Medikamentenbehälter abgestimmt sein muss. Somit kann in einer Transportbox nur ein Medikamentenbehälter, beziehungsweise ein Satz von Medikamentenbehältern mit exakt zu der Länge der Erhöhung korrespondierenden Positionen der Endstopfen zuverlässig gesichert werden. Auch sind derartige Transportboxen groß und ineffizient, wenn damit kleine Stückzahlen von Medikamentenbehältern mit individuell unterschiedlichen Endstopfenpositionen entlang der Mittelachsen der Medikamentenbehälter gesichert werden sollen.

Die Abfüllung von Medikamentenbehältern ist systembedingt mit Lufteinschlüssen in den zu befüllenden Kammern verbunden. Insbesondere bei Doppelkammerkarpulen ist der Luftanteil am gesamten Befüllungsvolumen vergleichsweise groß. Derartige Lufteinschlüsse führen dazu, dass eine Kraft auf die die Kammern begrenzenden Stopfen, insbesondere den Endstopfen wirkt, wenn der Medikamentenbehälter und damit der Endstopfen mit einem Unter- oder Überdruck beaufschlagt wird. Somit besteht insbesondere bei einem mit Höhenänderungen verbundenen Transport von Medikamentenbehältern, insbesondere beim Lufttransport oder beim Transport in einer Bergregion die Gefahr, dass es zu einer Verlagerung des Endstopfens kommt. Der beim Transport auftretende Druckgradient zwischen einem verschlossenen Bereich des Medikamentenbehälters und der Außenluft kann also zu einer Verlagerung des Endstopfens insbesondere in Richtung eines proximalen Endes des Medikamentenbehälters führen. Dadurch wird ein medizinisch steriler Bereich verunreinigt oder zumindest der Gefahr einer Verunreinigung ausgesetzt.

Aufgabe der Erfindung ist es, einen Medikamentenbehälter, eine Verwendung eines Stopfensicherungsteils und ein Stopfensicherungsteil und zu schaffen, wobei die genannten Nachteile vermieden werden.

Die Aufgabe wird gelöst, indem die Gegenstände der unabhängigen Ansprüche geschaffen werden. Vorteilhafte Ausgestaltungen ergeben sich aus den Unteransprüchen.

Die Aufgabe wird insbesondere gelöst, indem ein Medikamentenbehälter, insbesondere eine Spritze oder Karpule, besonders bevorzugt eine Doppelkammerspritze oder Doppelkammerkarpule, mit einem Grundkörper geschaffen wird, wobei ein proximales Ende des Grundkörpers als Applikationsende ausgebildet ist, und wobei ein distales Ende des Grundkörpers als Ausbringende ausgebildet ist, wobei der Grundkörper einen Innenraum zumindest abschnittsweise umschließt, wobei der Grundkörper eine Mittelachse und eine proximale Öffnung aufweist, wobei in dem Innenraum ein Endstopfen derart angeordnet ist, dass der Endstopfen in dem Innenraum entlang der Mittelachse verlagerbar ist und einen ersten, in Bezug auf den Endstopfen distalen Bereich des Innenraums von einem zweiten, in Bezug auf den Endstopfen proximalen Bereich des Innenraums trennt. Der Medikamentenbehälter weist ein Stopfensicherungsteil auf, das zumindest teilweise durch die proximale Öffnung in den zweiten Bereich des Innenraums einführbar ist, wobei das Stopfensicherungsteil in dem in den Innenraum eingeführten Zustand näher an der proximalen Öffnung als der Endstopfen angeordnet und durch Form- und/oder Kraftschluss zumindest in einem Haltebereich lagefest in dem Innenraum gehalten ist. Das Stopfensicherungsteil weist eine Anschlagsstruktur auf, wobei das Stopfensicherungsteil im Bereich der Anschlagsstruktur zumindest eine radiale Ausdehnung - in dem eingeführten Zustand senkrecht zu der Mittelachse - aufweist, die zumindest größer als eine minimale radiale Ausdehnung der proximalen Öffnung des Grundkörpers ist, sodass eine Einführtiefe des Stopfensicherungsteils in den Medikamentenbehälter durch Anschlag der Anschlagsstruktur an dem Medikamentenbehälter begrenzt ist. Außerdem ist das Stopfensicherungsteil luftdurchlässig ausgebildet. Dabei weist der Grundkörper vorzugsweise eine rotationssymmetrische, besonders bevorzugt zylindrische Form auf und umschließt den Innenraum, der zumindest teilweise eingerichtet ist, um mit einem medizinischen Wirkstoff und/oder Hilfsstoff befüllt zu werden. Zusammen mit dem Endstopfen und gegebenenfalls weiteren Stopfen oder anderen Verschlusselementen schließt der Grundkörper zumindest einen Teil des Innenraums komplett ein. Der Grundkörper weist außerdem vorzugsweise zumindest eine weitere, verschließbare Öffnung auf. Besonders bevorzugt weist der Grundkörper jedoch ausschließlich die proximale Öffnung und genau eine weitere, verschließbare, distale Öffnung auf. Im Folgenden wird davon ausgegangen, dass alle weiteren verschließbaren Öffnungen verschlossen sind.

Bei einem derartigen Medikamentenbehälter ist der Endstopfen sicher in dem Medikamentenbehälter gehalten. Eine Verlagerung des Endstopfens aus dem Medikamentenbehälter durch die proximale Öffnung ist durch das Stopfensicherungsteil verhinderbar und - im eingeführten Zustand - verhindert. Außerdem ist das Stopfensicherungsteil eines derartigen Medikamentenbehälters auf einfache Weise, insbesondere manuell, ein- und ausführbar.

Indem der Endstopfen dicht an einer Innenwand des Innenraums anliegt, trennt er den ersten Bereich von dem zweiten Bereich. Aufgrund dieser Trennung ist eine Reinheit und/oder Sterilität des ersten Bereichs sichergestellt. Der erste Bereich bildet also bei bestimmungsgemäßer Anordnung des Endstopfens einen abgeschlossenen Raum. Vorzugsweise ist der erste Bereich mittels zumindest eines weiteren Stopfens - insbesondere eines Mittelstopfens - in zwei separate Kammern unterteilt. Der Medikamentenbehälter bildet somit ein Doppelkammersystem, insbesondere eine Doppelkammerkarpule oder eine Doppelkammerspritze.

Der erste Bereich ist eingerichtet, einen medizinischen Wirk- und/oder Hilfsstoff aufzunehmen. Außerdem sind der Grundkörper, der Endstopfen und alle weiteren Öffnungen eingerichtet, den ersten Bereich gegenüber äußeren Verunreinigungen abzudichten. Der zweite Bereich dagegen ist nicht notwendigerweise abgedichtet. Vielmehr ist vorgesehen, dass eine Applikationsvorrichtung über den zweiten Bereich auf den Endstopfen einwirken kann, um den Endstopfen in Richtung des distalen Endes zu verlagern und somit einen in dem Medikamentenbehälter gelagerten Wirk- und/oder Hilfsstoff aus dem Medikamentenbehälter auszubringen. Der zweite Bereich steht also vorzugsweise über die proximale Öffnung des Grundkörpers mit der Umgebung des Medikamentenbehälters strömungstechnisch in Verbindung. Somit ist der zweite Bereich nicht steril und nicht vor Verunreinigungen geschützt.

Aufgrund der hier beschriebenen Anordnung des Endstopfen und des ersten Bereichs sowie des zweiten Bereichs ist der erste Bereich weiter von der proximalen Öffnung des Grundkörpers entfernt als der zweite Bereich.

Das Stopfensicherungsteil ist zumindest teilweise durch die proximale Öffnung in den zweiten Bereich des Innenraums einführbar, vorzugsweise eingeführt, und - im eingeführten Zustand - durch die proximale Öffnung aus dem zweiten Bereich des Innenraums ausführbar. Außerdem ist das Stopfensicherungsteil - im eingeführten Zustand - entlang der Mittelachse verlagerbar und vorzugsweise drehsymmetrisch, besonders bevorzugt rotationssymmetrisch zu der Mittelachse gelagert. Außerdem ist das Stopfensicherungsteil durch Form- und/oder Kraftschluss vorzugsweise mit der Innenwand oder einer Außenwand des Grundkörpers verbunden und somit lagefest in dem Innenraum gehalten. Vorzugsweise ist das Stopfensicherungsteil alternativ oder zusätzlich durch Form- und/oder Kraftschluss mit einer äußeren Struktur und/oder einem in den Grundkörper eingreifenden Element, insbesondere einem Transportbehälter, lagefest in dem Innenraum gehalten.

Gemäß einer Weiterbildung der Erfindung ist vorgesehen, dass das Stopfensicherungsteil derart lagefest gehalten und/oder derart relativ zu dem Endstopfen angeordnet ist, dass eine insbesondere druckbedingte Verlagerung des Endstopfens in proximaler Richtung durch das Stopfensicherungsteil begrenzt ist. Insbesondere ist also eine aus dem Medikamentenbehälter herausführende Verlagerung des Endstopfens verhindert und durch Anschlag des proximalen Endes des Endstopfens an dem distalen Ende des Stopfensicherungsteils begrenzt.

Besonders bevorzugt ist vorgesehen, dass das Stopfensicherungsteil im eingeführten Zustand unmittelbar an den Endstopfen angrenzt, sodass jegliche Verlagerung des Endstopfens in Richtung der proximalen Öffnung verhindert ist.

Vorzugsweise ist so also eine eine Sterilität des ersten Bereichs des Innenraums verletzende Verlagerung des Endstopfens in Richtung der proximalen Öffnung aufgrund des lagefest gehaltenen Stopfensicherungsteils verhindert. Die Sterilität des ersten Bereichs des Innenraums ist insbesondere dann gefährdet, wenn der Endstopfen komplett in den zweiten Bereich des Innenraums verlagert ist. Vorzugsweise ist die Verlagerung des Endstopfens in proximaler Richtung durch das Stopfensicherungsteil also derart begrenzt, dass der Endstopfen nicht vollständig in den zweiten Bereich verlagerbar ist. Dies ist insbesondere dann gewährleistet, wenn das distale Ende des Stopfensicherungsteils in einem Abstand zu dem proximalen Ende des Endstopfens angeordnet ist, wobei dieser Abstand kleiner ist als die axiale Länge des Endstopfens. Vorteilhafterweise ist so die Sterilität des ersten Bereichs besonders zuverlässig sichergestellt. Es ist aber auch möglich, dass das proximale Ende des Endstopfens an dem distalen Ende des Stopfensicherungsteils anliegt.

Gemäß einer Weiterbildung der Erfindung ist vorgesehen, dass das Stopfensicherungsteil mit der Innenwand des Grundkörpers formschlüssig und/oder kraftschlüssig zusammenwirkt. Ein derartiger Form- und/oder Kraftschluss ist besonders einfach zu schaffen und - verglichen mit einem Form- oder Kraftschluss zwischen Stopfensicherungsteil und einer Außenwand - störunanfälliger.

Gemäß einer Weiterbildung der Erfindung ist vorgesehen, dass das Stopfensicherungsteil ein elastisches Haltemittel mit einer vorzugsweise zumindest radial zur Mittelachse wirkenden Elastizität aufweist, wobei das elastische Haltemittel eingerichtet ist, um das Stopfensicherungsteil - in dessen in den Innenraum eingeführten Zustand - an dem Grundkörper, vorzugsweise an der Innenwand des Grundkörpers, kraftschlüssig zu halten. Mittels eines solchen elastischen Haltemittels ist das Stopfensicherungsteil zumindest in dem Haltebereich lagefest in dem Innenraum kraftschlüssig gehalten. Vorzugsweise ist mittels des elastischen Haltemittels auch ein Formschluss ermöglicht, indem das elastische Haltemittel durch eine radiale Verjüngung des zweiten Bereichs hindurch geführt wird und sich hinter der radialen Verjüngung aufgrund seiner Elastizität aufweitet und damit die Verjüngung hintergreift. Der Medikamentenbehälter mit einem solchen Stopfensicherungsteil hat den Vorteil, dass die Sicherung des Endstopfens mittels des elastischen Haltemittels einfach handhabbar ist und das Ein- und Ausführen eines solchen Stopfensicherungsteils in und aus dem zweiten Bereich somit besonders einfach, insbesondere manuell und schnell durchführbar ist.

Gemäß einer bevorzugten Weiterbildung ist vorgesehen, dass das elastische Haltemittel eine Feder, und/oder eine federartige Struktur, und/oder ein federartiges Element, und/oder ein vulkanisiertes Elastomer, vorzugsweise Gummi, und/oder eine Materialschwächungszone, vorzugsweise eine Aussparung, in welche angrenzendes Material, vorzugsweise - senkrecht zu der Mittelachse - außen angrenzendes Material, elastisch hinein verformbar ist, aufweist. Unter einer Materialschwächungszone wird hier ein Bereich verstanden, der ein Material aufweist, welches relativ zu dem die Materialschwächungszone umgebenden Material bei Krafteinwirkung nachgiebig und/oder komprimierbar ist. Die Materialschwächungszone wird also bei einer Krafteinwirkung stärker komprimiert als das die Materialschwächungszone umgebende Material. Alternativ oder zusätzlich weist die Materialschwächungszone eine Flüssigkeit und/oder ein Gas, insbesondere Luft auf, wobei das Gas und/oder die Flüssigkeit der Materialschwächungszone bei Krafteinwirkung aus der Materialschwächungszone heraus verdrängbar ist. Vorzugsweise weist das elastische Haltemittel eine Aussparung auf. Das Stopfensicherungsteil mit einem derartigen elastischen Haltemittel ist besonders kostengünstig und/oder einstückig und/oder materialeinheitlich herstellbar.

Gemäß einer Weiterbildung der Erfindung ist vorgesehen, dass das Stopfensicherungsteil zumindest in einem distalen Bereich des Stopfensicherungsteils einen runden, kreuzförmigen oder i-förmigen Querschnitt aufweist. Mittels eines derartigen Querschnitts in dem distalen Bereich des Stopfensicherungsteils, also dem Bereich, der beim Einführen des Stopfensicherungsteils in den zweiten Bereich des Medikamentenbehälters vorauseilt, ist eine besonders günstige Kräfteverteilung zur Realisierung des Kraftschlusses gegeben. So stützen sich in Bezug auf die Mittelachse gegenüberliegenden Stege, welche den kreuzförmigen oder i-förmigen Querschnitt bilden, und/oder die in Bezug auf die Mittelachse gegenüberliegenden Abschnitte, welche den runden Querschnitt bilden, sich vorzugsweise exakt gegeneinander ab, sofern das elastische Haltemittel - im eingeführten Zustand des Stopfensicherungsteils - in diesem distalen Bereich angeordnet und komprimiert ist. Somit sind die den Kraftschluss bildenden, radial wirkenden Kräfte lokal maximiert und davon abweichende Kraftkomponenten vermieden. Weiterhin ist mittels eines derartigen Querschnitts vorzugsweise sichergestellt, dass das Stopfensicherungsteil zumindest in dem distalen Bereich, bevorzugt in dem gesamten in den zweiten Bereich eindringenden Bereich des Stopfensicherungsteils luftdurchlässig ist, sodass beim Einführen des Stopfensicherungsteils in den Medikamentenbehälter ein dichter Abschluss des Bereichs zwischen dem Stopfensicherungsteil und dem Endstopfen vermieden wird. Somit wird vermieden, dass der Druck, insbesondere der Luftdruck zwischen dem Stopfensicherungsteil und dem Endstopfen, insbesondere während des Einführens oder des Ausführens des Stopfensicherungsteils, erhöht oder verringert ist. Auch eine unbeabsichtigte Verlagerung des Endstopfens in distale und/oder proximale Richtung wird so vermieden.

Gemäß einer Weiterbildung der Erfindung ist vorgesehen, dass das Stopfensicherungsteil im Bereich der Anschlagsstruktur zumindest eine weitere radiale Ausdehnung - in dem eingeführten Zustand senkrecht zu der Mittelachse - aufweist, die größer als eine maximale radiale Ausdehnung der proximalen Öffnung des Grundkörpers, sodass eine Einführtiefe des Stopfensicherungsteil in den Medikamentenbehälter durch Anschlag der Anschlagstruktur an dem Medikamentenbehälter begrenzt ist. Die Anschlagstruktur ist vorzugsweise im Randbereich des Stopfensicherungsteils, also - im eingeführten Zustand - in einem radialen Abstand zu der Mittelachse des Grundkörpers, insbesondere an einer Mantelfläche des Stopfensicherungsteils angeordnet. Vorzugsweise ist die Anschlagsstruktur als Verdickung oder Vorsprung gegenüber der Mantelfläche ausgebildet. Besonders bevorzugt ist die Anschlagsstruktur einstückig und/oder materialeinheitlich an dem Stopfensicherungsteil ausgebildet.

Ist die zumindest eine radiale Ausdehnung des Stopfensicherungsteils zumindest größer als die minimale radiale Ausdehnung der proximalen Öffnung des Grundkörpers, so gibt es zumindest eine Rotationsstellung - um die Mittelachse -, in welcher die Anschlagsstruktur - in dem eingeführten Zustand - an dem proximalen Ende des Medikamentenbehälters anschlägt und somit ein weiteres Eindringen des Stopfensicherungsteils in den Medikamentenbehälter verhindert. Vorzugsweise ist aber die zumindest eine radiale Ausdehnung des Stopfensicherungsteils größer als die maximale radiale Ausdehnung der proximalen Öffnung des Grundkörpers, sodass die Einführtiefe in jeder Rotationsstellung des Stopfensicherungsteils durch Anschlag der Anschlagsstruktur an dem proximalen Ende des Medikamentenbehälters begrenzt ist.

In jedem Fall ist die Anschlagsstruktur in einem axialen Abstand zum distalen Ende des Stopfensicherungsteils angeordnet, der kleiner oder gleich ist als/wie ein axialer Abstand zwischen dem proximalen Ende des Endstopfens und der proximalen Öffnung des Grundkörpers. Ein solcher Medikamentenbehälter hat den Vorteil, dass der darin angeordnete Endstopfen nicht, insbesondere nicht mittels des Stopfensicherungsteils verlagert werden kann. Somit ist die Sicherheit des Medikamentenbehälters und eines darin abgefüllten medizinischen Wirk- und/oder Hilfsstoff erhöht.

Gemäß einer Weiterbildung der Erfindung ist vorgesehen, dass an einem - in dem eingeführten Zustand des Stopfensicherungsteils - proximalen Ende des Stopfensicherungsteils ein Griffbereich ausgebildet ist, der eine Hinterschnittstruktur und/oder ein griffiges Material und/oder eine raue Oberflächenstruktur aufweist. Unter einem griffigen Material ist hier insbesondere ein rutschfestes Material, und/oder ein Material mit - relativ zu einem anderen Material des Stopfensicherungsteils - erhöhter Haftreibung, und/oder ein Gummi, und/oder ein gummiartiges Material zu verstehen. Mittels eines solchen Griffbereichs ist die Handhabbarkeit des Medikamentenbehälters verbessert. Das Stopfensicherungsteil des Medikamentenbehälters ist somit besonders einfach und schnell, insbesondere manuell ein- und ausführbar.

Gemäß einer Weiterbildung der Erfindung ist vorgesehen, dass der Medikamentenbehälter einen Sicherheitsindikator, vorzugsweise ein Etikett oder einen Schrumpfschlauch aufweist. Dabei ist vorgesehen, dass der Sicherheitsindikator - in dem eingeführten Zustand des Stopfensicherungsteil - in einem Bereich, in dem das Stopfensicherungsteil an den Grundkörper angrenzt, derart an dem Grundkörper und an dem Stopfensicherungsteil angeordnet, vorzugsweise befestigt ist, dass der Sicherheitsindikator durch eine - insbesondere sterilitätsverletzende - Verlagerung des Stopfensicherungsteils veränderbar, vorzugsweise beschädigbar oder zumindest teilweise entfernbar ist. Der Sicherheitsindikator weist vorzugsweise eine Sollbruchstelle, insbesondere eine Perforation auf. Mittels eines derartigen Sicherheitsindikators kann vorzugsweise visuell erkannt werden, ob eine Verlagerung des Stopfensicherungsteils stattgefunden hat. Insbesondere bei druckbedingten Verlagerungen, wie sie auf Transportwegen stattfinden, ist eine sterilitätsverletzende Verlagerung des Endstopfens und/oder des Stopfensicherungsteils oftmals im Nachhinein nicht feststellbar, da der Endstopfen wieder in seiner Ausgangslage zurückverlagert wird, wenn der Außenluftdruck wieder auf sein Ausgangsniveau zurückgeht. Mittels eines Sicherheitsindikators der hier beschriebenen Art ist jedoch vorteilhafterweise auch eine nur temporäre Verlagerung des Stopfensicherungsteils und/oder des Endstopfens nachträglich feststellbar. Der hier beschriebene Sicherheitsindikator wird bei einer - insbesondere sterilitätsverletzenden - Verlagerung des Stopfensicherungsteils derart irreversibel manipuliert, dass er bei einer Rückverlagerung des Endstopfens in seine Ausgangsposition nicht in seinen Ausgangszustand zurückkehrt. Somit bleibt auch eine temporäre Verlagerung vorteilhafterweise nicht unbemerkt. Damit ist die Sicherheit des Medikamentenbehälters, insbesondere eines in dem Medikamentenbehälter abgefüllten Medikaments, erhöht. Bei dem hier beschriebenen Ausführungsbeispiel eines Medikamentenbehälters mit einem Sicherheitsindikator ist das Stopfensicherungsteil im eingeführten Zustand vorzugsweise unmittelbar angrenzend an den Endstopfen angeordnet, sodass mittels des Sicherheitsindikators besonders zuverlässig auf eine Verlagerung des Endstopfens, zumindest in proximaler Richtung, geschlossen werden kann.

Gemäß einer Weiterbildung der Erfindung ist vorgesehen, dass das Stopfensicherungsteil Polypropylen, Polystyrol und/oder wenigstens ein Harz aufweist. Vorzugsweise ist das Stopfensicherungsteil in einem Kunststoffspritzgussverfahren gefertigt. Ein Medikamentenbehälter mit einem derartigen Stopfensicherungsteil hat den Vorteil, dass er besonders kostengünstig ist.

Gemäß einer Weiterbildung der Erfindung ist vorgesehen, dass das Stopfensicherungsteil einen maschinell, vorzugsweise berührungslos, besonders bevorzugt elektromagnetisch auslesbaren Informationsträger, insbesondere einen RFID-Chip aufweist. Mittels eines derartigen Informationsträgers ist es auf besonders kostengünstige Weise möglich, inhaltsspezifische und/oder transportspezifische und/oder herkunftsspezifische Informationen zu dem Medikamentenbehälter und/oder dessen Inhalt für den Medikamentenbehälter individualisiert zu erfassen und zu speichern. Somit sind derartige Informationen schneller speicher- und abrufbar sowie besser dem Medikamentenbehälter und/oder dessen Inhalt zuordenbar.

Gemäß einer Weiterbildung der Erfindung ist vorgesehen, dass das Stopfensicherungsteil derart geformt ist, dass es - in dem eingeführten Zustand - keine äußere Umfangsfläche des Medikamentenbehälters überdeckt. Somit ist eine an der äußeren Umfangsfläche angebrachte Information erkennbar und die Handhabung des Medikamentenbehälters verbessert.

Gemäß einem bevorzugten Ausführungsbeispiel ist vorgesehen, dass das Stopfensicherungsteil des Medikamentenbehälters farbig, insbesondere einfarbig oder mehrfarbig, weiß oder schwarz ausgebildet und/oder mit einem Muster oder Zeichen versehen ist. Diese Ausgestaltung des Stopfensicherungsteil dient vorzugsweise einer Kodierung, mittels der einem derartigen optischen Kennzeichen - also Farbe, Muster und/oder Zeichen - zumindest eine Information, insbesondere eine das Medikament, eine Medikamentendosierung und/oder eine Medikamentenkonfiguration betreffende Information zugeordnet ist. Unter einer Medikamentenkonfiguration wird hier insbesondere eine Zusammensetzung verschiedener Wirk- und/oder Hilfsstoffe verstanden.

Es wird auch ein nicht zur Erfindung gehörender Satz oder eine Zusammenstellung von Medikamentenbehältern gemäß einem der zuvor beschriebenen Ausführungsbeispiele beschrieben, wobei jedem Medikamentenbehälter jeweils ein Stopfensicherungsteil zugeordnet ist, das farbig, insbesondere einfarbig oder mehrfarbig, weiß oder schwarz, und/oder mit einem Muster oder Zeichen versehen ist.

Durch die farbige Ausgestaltung der Stopfensicherungsteile und/oder deren Ausstattung mit einem Muster oder Zeichen sind die verschiedenen Medikamentenbehälter des Satzes oder der Zusammenstellung optisch gekennzeichnet, wobei der jeweiligen Kennzeichnung, das heißt der Farbe, dem Muster und/oder dem Zeichen, jeweils eine Information über den Medikamentenbehälter zugeordnet ist, beispielsweise eine Information über ein in dem Medikamentenbehälter angeordnetes Medikament, eine Medikamentendosierung und/oder eine Medikamentenkonfiguration. Abhängig insbesondere von dem in dem Medikamentenbehälter angeordneten Medikament und/oder der Medikamentenkonfiguration kann die Position des Endstopfens variieren. Entsprechend sind auch die dem Medikamentenbehälter jeweils zugeordneten Stopfensicherungsteile bevorzugt verschieden lang ausgebildet, um jeweils möglichst nah durch die proximale Öffnung hindurch in den zweiten Bereich des Innenraums an den Endstopfen in der jeweiligen Position heranreichen zu können. Auch diese verschiedene Länge der Stopfensicherungsteile ist bevorzugt - alternativ oder zusätzlich - durch die Farbe, das Muster und/oder das Zeichen kodiert.

Insbesondere bei einem Satz von Medikamentenbehältern mit jeweils verschiedenen Stopfensicherungsteilen ist eine derart kodierte Information durch optische Kontrolle erfassbar und dem jeweiligen Medikamentenbehälter zuordenbar. Besonders bevorzugt ist auch der Länge - entlang der Mittelachse - des Stopfensicherungsteils eine derartige Information zugeordnet.

Besonders bevorzugt ist bei dem Satz von Medikamentenbehältern die Länge jedes Stopfensicherungsteils auf die Position des entsprechenden Endstopfens in dem Medikamentenbehälter derart abgestimmt, dass die jeweiligen Stopfensicherungsteile unmittelbar an den entsprechenden Endstopfen angrenzen. Somit ist es möglich, bei einem Satz von Medikamentenbehältern mit gleichen Grundkörpern aber unterschiedlichen Endstopfenposition jeden einzelnen Endstopfen zuverlässig und individuell abgestimmt zu sichern.

Es wird in entsprechender Weise auch ein nicht zur Erfindung gehörender Satz oder eine Zusammenstellung von Stopfensicherungsteilen beschrieben, die farbig, insbesondere einfarbig oder mehrfarbig, weiß oder schwarz ausgebildet, und/oder mit einem Muster oder Zeichen versehen sind. Dabei unterscheiden sich wenigstens zwei der Stopfensicherungsteile bezüglich der Farbe, des Musters und/oder des Zeichens voneinander, wobei den verschiedenen Farben, Mustern und/oder Zeichen verschiedene Informationen - insbesondere gemäß einer Kodierung - zugeordnet sind, wobei die Informationen bevorzugt ein in einem mit dem jeweiligen Stopfensicherungsteil zu sichernden Medikamentenbehälter angeordnetes Medikament, eine Medikamentendosierung und/oder eine Medikamentenkonfiguration betreffen.

Es wird auch ein nicht zur Erfindung gehörender Transportbehälter für zumindest einen Medikamentenbehälter beschrieben, der zumindest eine Aufnahme für den zumindest einen Medikamentenbehälter aufweist, wobei die Aufnahme eine Länge aufweist, die einer axialen Länge - entlang der Mittelachse - des Medikamentenbehälters mit eingeführtem Stopfensicherungsteil entspricht. Somit ist der Medikamentenbehälter zumindest in Richtung seiner Länge spielfrei in der Aufnahme des Transportbehälters lagerbar. Vorzugsweise ist ein in dem Transportbehälter gelagerter Medikamentenbehälter auch senkrecht zu der Mittelachse spielfrei lagerbar. Sofern der Transportbehälter mehrere Aufnahmen aufweist, ist es vorteilhafterweise möglich, mehrere Medikamentenbehälter, auch mit unterschiedlichen Endstopfenposition - entlang der Mittelachse - und entsprechend darauf abgestimmten Stopfensicherungsteilen, zuverlässig zu lagern, ohne dass die Sicherheit des Medikamentenbehälters und/oder die Sterilität indem ersten Bereich gefährdet ist. Insbesondere kann jede Aufnahme des Medikamentenbehälters gleich lang sein. Durch Anpassung der Stopfensicherungsteile ist es so möglich, einen Satz von Medikamentenbehältern in dem Transportbehälter anzuordnen, wobei der Satz zumindest zwei Medikamentenbehälter aufweist, welche voneinander verschiedene Endstopfenpositionen aufweisen.

Die Aufgabe wird insbesondere auch gelöst, indem eine Verwendung eines Stopfensicherungsteils in einem Medikamentenbehälter zum Sichern eines Endstopfens geschaffen wird. Der Medikamentenbehälter ist vorzugsweise nach einem der vorhergehenden Ausführungsbeispiele ausgebildet. Dabei ist vorgesehen, dass der Medikamentenbehälter einen Grundkörper, der einen Innenraum zumindest abschnittsweise umschließt, eine Mittelachse und eine proximale Öffnung aufweist, wobei in dem Innenraum ein Endstopfen derart angeordnet ist, dass der Endstopfen in dem Innenraum entlang der Mittelachse verlagerbar ist und einen ersten, in Bezug auf den Endstopfen distalen Bereich des Innenraums von einem zweiten, in Bezug auf den Endstopfen proximalen Bereich des Innenraums trennt. Die Verwendung des Stopfensicherungsteils zeichnet sich dadurch aus, dass das Stopfensicherungsteil durch die proximale Öffnung in den zweiten Bereich derart eingeführt wird, dass es näher als der Endstopfen an der proximalen Öffnung gelagert ist, wobei das Stopfensicherungsteil mittels Form- und/oder Kraftschluss zumindest in einem Haltebereich des zweiten proximalen Bereichs des Medikamentenbehälters gehalten wird. Somit ergeben sich die bereits zuvor in Bezug auf den Medikamentenbehälter genannten Vorteile. Insbesondere ist bei einer derartigen Verwendung des Stopfensicherungsteils in einem Medikamentenbehälter der Endstopfen besonders zuverlässig gehalten.

Gemäß einer Ausführungsform der Verwendung ist vorgesehen, dass die Verlagerung des Endstopfens durch das Stopfensicherungsteil begrenzt wird, vorzugsweise derart, dass die Sterilität des ersten Bereichs des Innenraums sichergestellt ist. Die Verlagerung des Endstopfens ist insbesondere durch Anschlag des proximalen Endes des Endstopfens an dem distalen Ende des Stopfensicherungsteils in proximaler Richtung begrenzt.

Es wird auch ein nicht zur Erfindung gehörendes Stopfensicherungsteil beschrieben, welches zur Verwendung in einem Medikamentenbehälter nach einem der zuvor beschriebenen Ausführungsbeispiele eingerichtet ist. Dabei ergeben sich die jeweils zuvor genannten Vorteile.

Insgesamt zeigt sich, dass mit dem hier dargestellten Medikamentenbehälter, dem Transportbehälter, dem Stopfensicherungsteil und der Verwendung des Stopfensicherungsteils in einem Medikamentenbehälter ein Endstopfen auf besonders zuverlässige Art in einem Medikamentenbehälter gehalten ist.

Die Erfindung wird im Folgenden anhand der Zeichnungen näher erläutert. Dabei zeigen:
- Figur 1: eine Seitenansicht eines Ausführungsbeispiel eines Medikamentenbehälters,
- Figur 2: eine vergrößerte Darstellung des proximalen Endes des Medikamentenbehälter gemäß dem Ausführungsbeispiel nach Figur 1, und
- Figur 3: eine perspektivische Darstellung eines Stopfensicherungsteils gemäß den Figuren 1 und 2.

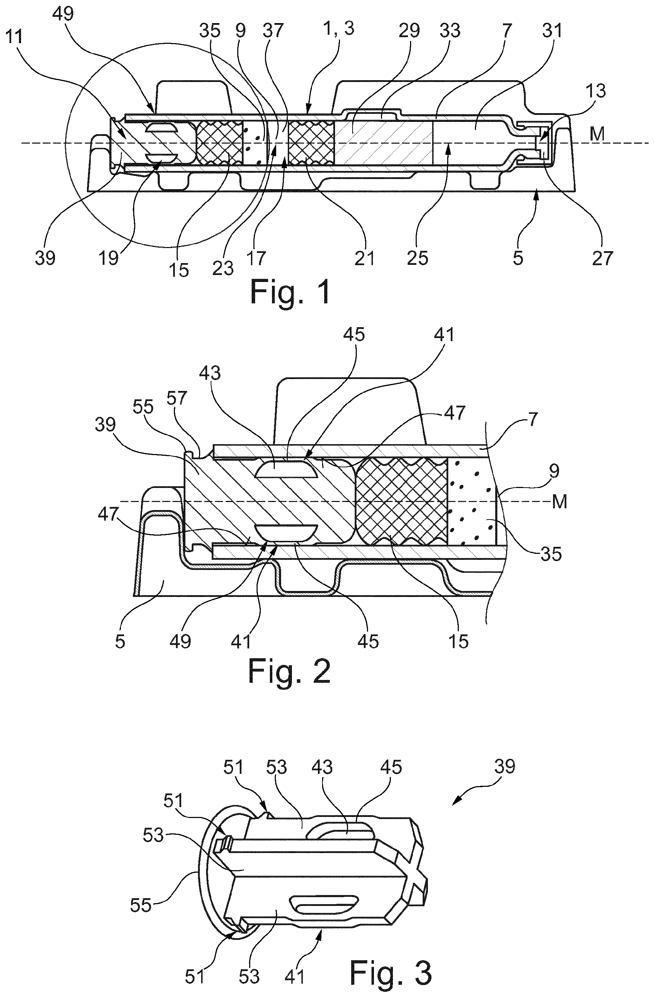

Figur 1 zeigt ein Ausführungsbeispiel eines Medikamentenbehälters 1, hier einer Doppelkammerkarpule 3, in einer Längsschnittdarstellung. Der Medikamentenbehälter 1 ist hier in einem Transportbehälter 5 entlang einer Mittelachse M spielfrei gelagert. Der Medikamentenbehälter 1 weist einen Grundkörper 7, der einen Innenraum 9 im Wesentlichen zylindermantelförmig umschließt, die Mittelachse M und eine proximale Öffnung 11 sowie eine distale Öffnung 13 auf. In dem Innenraum 9 des Grundkörpers 7 ist ein Endstopfen 15 derart angeordnet, dass dieser Endstopfen 15 in dem Innenraum 9 entlang der Mittelachse M verlagerbar ist und einen ersten, in Bezug auf den Endstopfen distalen Bereich 17 von einem zweiten, in Bezug auf den Endstopfen proximalen Bereich 19 des Innenraums 9 trennt. Der erste Bereich 17 ist hier mittels eines Mittelstopfens 21 in eine erste Kammer 23 und eine zweite Kammer 25 geteilt.

Die distale Öffnung 13 ist mit einem Verschlusselement 27 verschlossen, sodass die zweite Kammer 25 einen geschlossenen Raum bildet, welcher von dem Grundkörper 7, dem Mittelstopfen 21 und dem Verschlusselement 27 begrenzt ist. Innerhalb der zweiten Kammer 25 ist hier ein Lyophilisat 29 gelagert, welches im Wesentlichen von Luft 31 umgeben ist. Im Bereich der zweiten Kammer 25 ist hier außerdem ein Bypass 33 im Grundkörper 7 ausgebildet. Dieser ermöglicht nach einer Verlagerung des Mittelstopfens 21 in Richtung der distalen Öffnung entlang der Mittelachse M eine Durchmischung der Inhalte der beiden Kammern 23, 25.

Die erste Kammer 23 weist hier ein Lösungsmittel 35 und eine Luftblase 37 auf. Auch die erste Kammer bildet einen geschlossenen Raum, welcher durch den Mittelstopfen 21, den Grundkörper 7 und den Endstopfen 15 begrenzt ist. Nach Öffnen der distalen Öffnung 13 ist es möglich, mittels einer hier nicht dargestellten Applikationsvorrichtung den Endstopfen 15 und damit zugleich den Mittelstopfen 21 in Richtung der distalen Öffnung 13 zu verlagern. Gelangt der Mittelstopfen 21 in den axialen Abschnitt des Bypasses 33, wird der Inhalt der ersten Kammer 23, also insbesondere das Lösungsmittel 35 in die zweite Kammer 25 überführt. Somit ist die Durchmischung der beiden Kammerinhalte möglich. Wird der Endstopfen 15 mittels der Applikationsvorrichtung weiter in Richtung der distalen Öffnung 13 verlagert, wird der durchmischte Inhalt der zweiten Kammer 25 über die distale Öffnung 13 aus dem Innenraum 9 ausgebracht.

In dem hier dargestellten Ausführungsbeispiel ist ein Stopfensicherungsteil 39 in dem zweiten Bereich 19 derart angeordnet, dass der Endstopfen 15 mit seinem der proximalen Öffnung 11 zugewandten Ende unmittelbar an das der distalen Öffnung 13 zugewandte Ende des Stopfensicherungsteils 39 angrenzt. Somit ist eine Einwirkung über die proximale Öffnung 11 auf den Endstopfen 15, insbesondere mittels einer Applikationsvorrichtung, hier nicht möglich, und der Endstopfen damit vor einer derartigen Einwirkung und einer damit verbundenen Verlagerung in Richtung der distalen Öffnung 13 geschützt.

Figur 2 zeigt eine vergrößerte Darstellung des proximalen Bereichs des Medikamentenbehälters 1 gemäß Figur 1. Gleiche und funktionsgleiche Elemente sind mit gleichen Bezugszeichen versehen, sodass insofern auf die vorangegangene Beschreibung verwiesen wird.

In Figur 2 wird deutlich, dass das Stopfensicherungsteil 39 mittels wenigstens eines elastischen Haltemittels 41, hier einer Mehrzahl elastischer Haltemittel 41 kraftschlüssig in dem zweiten Bereich 19 des Innenraums 9 gehalten ist. Das wenigstens eine elastische Haltemittel 41 weist eine Materialschwächungszone 43 auf, welche hier als Aussparung ausgebildet ist. An die Materialschwächungszone 43 schließt sich außen - in radialer Richtung gesehen - eine stegförmige Struktur 45 des Stopfensicherungsteils 39 an, welche elastische Eigenschaften aufweist.

In dem hier dargestellten, eingeführten Zustand des Stopfensicherungsteils 39 ist die stegförmige Struktur 45 in die Materialschwächungszone 43 elastisch hinein verformt, sodass eine Federkraft von innen auf die Innenwand 47 des Grundkörpers 7 wirkt und das Stopfensicherungsteil 39 durch Kraftschluss in einem Haltebereich 49 des Innenraums 9 hält.

Figur 3 zeigt eine perspektivische Darstellung des Stopfensicherungsteils 39 gemäß den Figuren 1 und 2. In dieser Darstellung zeigt sich, dass das Stopfensicherungsteil 39 hier einen kreuzförmigen Querschnitt mit zwei einander diametral gegenüberliegenden elastischen Haltemitteln 41 aufweist. Dieser kreuzförmige Querschnitt erstreckt sich ausgehend von dem distalen Ende über einen Großteil des Stopfensicherungsteils 39, insbesondere über den distalen Bereich, der im eingeführten Zustand im zweiten Bereich 19 des Grundkörpers 7 angeordnet ist.

Das Stopfensicherungsteil 39 befindet sich in Figur 3 nicht in dem eingeführten Zustand, sodass das elastische Haltemittel 41 entspannt ist, wobei und keine mechanische Spannungsenergie darin gespeichert ist. Somit springt die stegförmige Struktur 45 in radialer Richtung, also senkrecht zur Mittelachse M, gegenüber den der stegförmigen Struktur 45 benachbarten Bereichen hervor. Durch Einführen dieses Stopfensicherungsteils 39 in einen Medikamentenbehälter 1 mit einem zylindrischen Innenraum 9 werden diese stegförmigen Strukturen 45 radial nach innen - in Richtung der Mittelachse M - in die Materialschwächungszone 43 hinein verformt.

Weiterhin ist in Figur 3 erkennbar, dass das Stopfensicherungsteil 39 in seinem hinteren distalen Bereich, welcher - im eingeführten Zustand des Stopfensicherungsteils 39 - der distalen Öffnung 13 abgewandt ist, eine Anschlagsstruktur 51 aufweist. In Figur 3 sind insbesondere drei Anschlagsstrukturen 51 dargestellt, wobei eine vierte Anschlagsstruktur 51 auf der dem Betrachter abgewandten Seite angeordnet ist. Jede der Anschlagsstrukturen 51 ist hier als hervorspringende Stufe auf einem von vier Stegen 53, welche senkrecht aufeinanderstehen und den kreuzförmigen Querschnitt bilden, ausgebildet. Jeweils zwei der Anschlagsstrukturen 51, welche gegenüberliegend angeordnet sind, spannen eine Breite auf, welche größer als ein Durchmesser der proximalen Öffnung 11 des Medikamentenbehälters 1 ist. Bei dem hier dargestellten Stopfensicherungsteil 39 weist insbesondere jede Anschlagsstruktur 51 eine radiale Ausdehnung - in dem eingeführten Zustand senkrecht zu der Mittelachse 11 - auf, welche größer ist als ein Radius der proximalen Öffnung 11 des Medikamentenbehälters 1. Somit wird verhindert, dass das Stopfensicherungsteil 39 beim Einführen in den Medikamentenbehälter 1 zu weit in den Medikamentenbehälter 1 eingeführt wird. Damit ist verhindert, dass der Endstopfen 15 aus seiner vorgesehenen Position mittels des Stopfensicherungsteils 39 in Richtung der distalen Öffnung 13 verlagert wird.

In einem besonders bevorzugten Ausführungsbeispiel des Stopfensicherungsteils 39 des Medikamentenbehälters 1 ist der Abstand der hervorspringenden Stufe der Anschlagsstruktur 51 zum distalen Ende des Stopfensicherungsteils 39 derart auf die axiale Position des Endstopfens 15 in dem Medikamentenbehälter 1 abgestimmt, dass das Stopfensicherungsteil 39 im eingeführten Zustand mit der Anschlagsstruktur 51 an dem proximalen Ende des Medikamentenbehälters 1 anschlägt, wobei das distale Ende des Stopfensicherungsteils 39 unmittelbar an das proximale Ende des Endstopfens 15 angrenzt, ohne dass der Endstopfen 15 durch das Einführen des Stopfensicherungsteils 39 aus seiner ursprünglichen Position verlagert wird.

Am proximalen Ende des Stopfensicherungsteils 39 ist außerdem ein Griffbereich 55 ausgebildet, welcher in Figur 3 besonders gut erkennbar ist. Der Griffbereich 55 wird hier durch eine runde Griffplatte gebildet, welche ausgehend vom proximalen Ende eine Hinterschnittstruktur 57 aufweist, die insbesondere in Figur 2 erkennbar ist. Somit kann das Stopfensicherungsteil 39 im eingeführten Zustand besonders einfach, insbesondere manuell gegriffen werden, um es aus dem Medikamentenbehälter 1 zu entnehmen.

Insgesamt zeigt sich, dass mit dem hier vorgeschlagenen Medikamentenbehälter 1, dem Stopfensicherungsteil 39 und der Verwendung des Stopfensicherungsteils 39 in dem Medikamentenbehälter 1 eine besonders zuverlässige und vielseitig einsetzbare und auf einen Medikamentenbehälter 1 individualisierbare Sicherung eines Endstopfens 15 in einem Medikamentenbehälter 1 geschaffen ist.

## Patentansprüche

1. Medikamentenbehälter (1) mit einem Grundkörper (7), wobei ein proximales Ende des Grundkörpers (7) als Applikationsende ausgebildet ist, und wobei ein distales Ende des Grundkörpers (7) als Ausbringende ausgebildet ist, wobei der Grundkörper (7) einen Innenraum (9) zumindest abschnittsweise umschließt, wobei der Grundkörper (7) eine Mittelachse (M) und eine proximale Öffnung (11) aufweist, wobei in dem Innenraum (9) ein Endstopfen (15) derart angeordnet ist, dass der Endstopfen (15) in dem Innenraum (9) entlang der Mittelachse (M) verlagerbar ist und einen ersten, in Bezug auf den Endstopfen (15) distalen Bereich (17) des Innenraums (9) von einem zweiten, in Bezug auf den Endstopfen (15) proximalen Bereich (19) des Innenraums (9) trennt, wobei der Medikamentenbehälter (1) ein Stopfensicherungsteil (39) aufweist, das zumindest teilweise durch die proximale Öffnung (11) in den zweiten Bereich (19) des Innenraums (9) einführbar ist, wobei das Stopfensicherungsteil (39) in dem in den Innenraum (9) eingeführten Zustand näher an der proximalen Öffnung (11) angeordnet ist als der Endstopfen (15), und durch Form- und/oder Kraftschluss zumindest in einem Haltebereich (49) lagefest in dem Innenraum (9) gehalten ist, wobei das Stopfensicherungsteil (39) eine Anschlagsstruktur (51) aufweist, wobei das Stopfensicherungsteil (39) im Bereich der Anschlagsstruktur (51) zumindest eine radiale Ausdehnung - in dem eingeführten Zustand senkrecht zu der Mittelachse (M) - aufweist, die zumindest größer als eine minimale radiale Ausdehnung der proximalen Öffnung (11) des Grundkörpers ist, sodass eine Einführtiefe des Stopfensicherungsteils (39) in den Medikamentenbehälter (1) durch Anschlag der Anschlagsstruktur (51) an dem Medikamentenbehälter (1) begrenzt ist, **dadurch gekennzeichnet, dass** das Stopfensicherungsteil (39) luftdurchlässig ausgebildet ist.

2. Medikamentenbehälter (1) nach Anspruch 1, **dadurch gekennzeichnet, dass** das Stopfensicherungsteil (39) derart lagefest gehalten und/oder derart relativ zu dem Endstopfen (15) angeordnet ist, dass eine Verlagerung des Endstopfens (15) in proximaler Richtung durch das Stopfensicherungsteil (39) begrenzt ist, wobei das Stopfensicherungsteil (39) die Verlagerung des Endstopfens (15) in proximaler Richtung durch Anschlag eines proximalen Endes des Endstopfens (15) an ein distales Ende des Stopfensicherungsteils (39) begrenzt.

3. Medikamentenbehälter (1) nach Anspruch 1 oder 2, **dadurch gekennzeichnet, dass** das Stopfensicherungsteil (39) mit einer Innenwand (47) des Grundkörpers (7) formschlüssig und/oder kraftschlüssig zusammenwirkt.

4. Medikamentenbehälter (1) nach einem der vorhergehenden Ansprüche, **dadurch gekennzeichnet, dass** das Stopfensicherungsteil (39) wenigstens ein elastisches Haltemittel (41) mit einer vorzugsweise zumindest radial zur Mittelachse (M) wirkenden Elastizität aufweist, wobei das elastische Haltemittel (41) eingerichtet ist, um das Stopfensicherungsteil (39) - in dessen in den Innenraum (9) eingeführten Zustand - an dem Grundkörper (7) kraftschlüssig zu halten.

5. Medikamentenbehälter (1) nach einem der vorhergehenden Ansprüche, **dadurch gekennzeichnet, dass** das elastische Haltemittel (41)
a) eine Feder, und/oder
b) eine federartige Struktur, und/oder
c) ein federartiges Element, und/oder
d) ein vulkanisiertes Elastomer, und/oder
e) eine Materialschwächungszone (43), in welche angrenzendes Material elastisch hinein verformbar ist,
aufweist.

6. Medikamentenbehälter (1) nach einem der vorhergehenden Ansprüche, **dadurch gekennzeichnet, dass** das Stopfensicherungsteil (39) zumindest in einem distalen Bereich des Stopfensicherungsteils (39) einen runden, kreuzförmigen oder I-förmigen Querschnitt aufweist.

7. Medikamentenbehälter (1) nach einem der vorhergehenden Ansprüche, **dadurch gekennzeichnet, dass** das Stopfensicherungsteil (39) im Bereich der Anschlagsstruktur (51) zumindest eine weitere radiale Ausdehnung - in dem eingeführten Zustand senkrecht zu der Mittelachse (M) - aufweist, die größer als eine maximale radiale Ausdehnung der proximalen Öffnung (11) des Grundkörpers ist, sodass eine Einführtiefe des Stopfensicherungsteils (39) in den Medikamentenbehälter (1) durch Anschlag der Anschlagsstruktur (51) an dem Medikamentenbehälter (1) begrenzt ist.

8. Medikamentenbehälter (1) nach einem der vorhergehenden Ansprüche, **dadurch gekennzeichnet, dass** an einem - in dem eingeführten Zustand - proximalen Ende des Stopfensicherungsteils (39) ein Griffbereich (55) ausgebildet ist, der
a) eine Hinterschnittstruktur (57), und/oder
b) ein griffiges Material, und/oder
c) eine raue Oberflächenstruktur
aufweist.

9. Medikamentenbehälter (1) nach einem der vorhergehenden Ansprüche, **dadurch gekennzeichnet, dass** der Medikamentenbehälter (1) einen Sicherheitsindikator oder einen Schrumpfschlauch, aufweist, der - in dem eingeführten Zustand des Stopfensicherungsteils (39) - in einem Bereich, in dem das Stopfensicherungsteil (39) an den Grundkörper (7) angrenzt, derart an dem Grundkörper (7) und an dem Stopfensicherungsteil (39) angeordnet ist, dass der Sicherheitsindikator durch eine Verlagerung des Stopfensicherungsteils (39) veränderbar ist.

10. Medikamentenbehälter (1) nach einem der vorhergehenden Ansprüche, **dadurch gekennzeichnet, dass** das Stopfensicherungsteil (39) Polypropylen, Polystyrol und/oder wenigstens ein Harz aufweist.

11. Medikamentenbehälter (1) nach einem der vorhergehenden Ansprüche, **dadurch gekennzeichnet, dass** das Stopfensicherungsteil (39) einen maschinell auslesbaren Informationsträger aufweist.

12. Medikamentenbehälter (1) nach einem der vorhergehenden Ansprüche, **dadurch gekennzeichnet, dass** das Stopfensicherungsteil (39) derart geformt ist, dass es - in dem eingeführten Zustand - keine äußere Umfangsfläche des Medikamentenbehälters (1) überdeckt.

13. Verwendung eines Stopfensicherungsteils (39) zum Sichern eines Endstopfens (15) in einem Medikamentenbehälter (1), nach einem der Ansprüche 1 bis 12, wobei der Medikamentenbehälter (1) einen Grundkörper (7), der einen Innenraum (9) zumindest abschnittsweise umschließt, eine Mittelachse (M) und eine proximale Öffnung (11) aufweist, wobei in dem Innenraum (9) ein Endstopfen (15) derart angeordnet ist, dass der Endstopfen (15) in dem Innenraum (9) entlang der Mittelachse (M) verlagerbar ist und einen ersten, in Bezug auf den Endstopfen (15) distalen Bereich (17) des Innenraums (9) von einem zweiten, in Bezug auf den Endstopfen (15) proximalen Bereich (19) des Innenraums (9) trennt, **dadurch gekennzeichnet, dass** das Stopfensicherungsteil (39) durch die proximale Öffnung (11) in den zweiten Bereich (19) derart eingeführt wird, dass es näher als der Endstopfen (15) an der proximalen Öffnung (11) gelagert ist, wobei das Stopfensicherungsteil (39) mittels Form- und/oder Kraftschluss zumindest in einem Haltebereich (49) des zweiten proximalen Bereichs des Medikamentenbehälters (1) lagefest gehalten wird.

14. Verwendung eines Stopfensicherungsteils (39) nach Anspruch 13, **dadurch gekennzeichnet, dass** die Verlagerung des Endstopfens (15) durch das Stopfensicherungsteil (39) begrenzt wird.

## Claims

1. Medication container (1) having a base body (7), wherein a proximal end of the base body (7) is adapted as an application end, and wherein a distal end of the base body (7) is adapted as a dispensing end, wherein the base body (7) at least sectionally encloses an interior space (9), wherein the base body (7) has a central axis (M) and a proximal opening (11), wherein an end stopper (15) is arranged in the interior space (9) such that the end stopper (15) is displaceable in the interior space (9) along the central axis (M) and separates a first region (17) of the interior space (9), distal with respect to the end stopper (15), from a second region (19) of the interior space (9), proximal with respect to the end stopper (15), wherein the medication container (1) has a stopper securing part (39) which is at least partially insertable through the proximal opening (11) into the second region (19) of the interior space (9), wherein the stopper securing part (39), in the state inserted into the interior space (9), is arranged closer to the proximal opening (11) than the end stopper (15), and is held positionally fixed in the interior space (9) by positive and/or force fit at least in a holding region (49), wherein the stopper securing part (39) has a stop structure (51), wherein the stopper securing part (39), in the region of the stop structure (51), has at least one radial extension - in the inserted state perpendicular to the central axis (M) - which is at least greater than a minimum radial extension of the proximal opening (11) of the base body, such that an insertion depth of the stopper securing part (39) into the medication container (1) is limited by abutment of the stop structure (51) against the medication container (1), **characterized in that** the stopper securing part (39) is adapted to be air-permeable.

2. Medication container (1) according to claim 1, **characterized in that** the stopper securing part (39) is held positionally fixed in such a way and/or arranged relative to the end stopper (15) in such a way that a displacement of the end stopper (15) in the proximal direction is limited by the stopper securing part (39), wherein the stopper securing part (39) limits the displacement of the end stopper (15) in the proximal direction by abutment of a proximal end of the end stopper (15) against a distal end of the stopper securing part (39).

3. Medication container (1) according to claim 1 or 2, **characterized in that** the stopper securing part (39) cooperates with an inner wall (47) of the base body (7) in a positive-fitting and/or force-fitting manner.

4. Medication container (1) according to one of the preceding claims, **characterized in that** the stopper securing part (39) has at least one elastic holding means (41) having an elasticity preferably acting at least radially with respect to the central axis (M), wherein the elastic holding means (41) is configured to hold the stopper securing part (39) - in its state inserted into the interior space (9) - on the base body (7) in a force-fitting manner.

5. Medication container (1) according to one of the preceding claims, **characterized in that** the elastic holding means (41) has
a) a spring, and/or
b) a spring-like structure, and/or
c) a spring-like element, and/or
d) a vulcanized elastomer, and/or
e) a material weakening zone (43), into which adjacent material is elastically deformable.

6. Medication container (1) according to one of the preceding claims, **characterized in that** the stopper securing part (39) has, at least in a distal region of the stopper securing part (39), a round, cross-shaped or I-shaped cross section.

7. Medication container (1) according to one of the preceding claims, **characterized in that** the stopper securing part (39), in the region of the stop structure (51), has at least one further radial extension - in the inserted state perpendicular to the central axis (M) - which is greater than a maximum radial extension of the proximal opening (11) of the base body, such that an insertion depth of the stopper securing part (39) into the medication container (1) is limited by abutment of the stop structure (51) against the medication container (1).

8. Medication container (1) according to one of the preceding claims, **characterized in that** a grip region (55) is adapted at an end of the stopper securing part (39) that is proximal in the inserted state, which has
a) an undercut structure (57), and/or
b) a grippy material, and/or
c) a rough surface structure.

9. Medication container (1) according to one of the preceding claims, **characterized in that** the medication container (1) has a security indicator or a shrink tube, which - in the inserted state of the stopper securing part (39) - is arranged on the base body (7) and on the stopper securing part (39), in a region in which the stopper securing part (39) adjoins the base body (7), in such a way that the security indicator is changeable by a displacement of the stopper securing part (39).

10. Medication container (1) according to one of the preceding claims, **characterized in that** the stopper securing part (39) has polypropylene, polystyrene and/or at least one resin.

11. Medication container (1) according to one of the preceding claims, **characterized in that** the stopper securing part (39) has a machine-readable information carrier.

12. Medication container (1) according to one of the preceding claims, **characterized in that** the stopper securing part (39) is shaped in such a way that it - in the inserted state - does not cover any outer circumferential surface of the medication container (1).

13. Use of a stopper securing part (39) for securing an end stopper (15) in a medication container (1), according to one of claims 1 to 12, wherein the medication container (1) has a base body (7), which at least sectionally encloses an interior space (9), a central axis (M) and a proximal opening (11), wherein an end stopper (15) is arranged in the interior space (9) in such a way that the end stopper (15) is displaceable in the interior space (9) along the central axis (M) and separates a first region (17) of the interior space (9), distal with respect to the end stopper (15), from a second region (19) of the interior space (9), proximal with respect to the end stopper (15), **characterized in that** the stopper securing part (39) is inserted through the proximal opening (11) into the second region (19) in such a way that it is mounted closer than the end stopper (15) to the proximal opening (11), wherein the stopper securing part (39) is held positionally fixed by means of positive and/or force fit at least in a holding region (49) of the second proximal region of the medication container (1).

14. Use of a stopper securing part (39) according to claim 13, **characterized in that** the displacement of the end stopper (15) is limited by the stopper securing part (39).

## Revendications

1. Récipient pour médicaments (1) muni d'un corps de base (7), dans lequel une extrémité proximale du corps de base (7) est conçue comme une extrémité d'application, et dans lequel une extrémité distale du corps de base (7) est conçue comme une extrémité de distribution, le corps de base (7) entourant au moins par endroits un espace interne (9), le corps de base (7) comportant un axe central (M) et une ouverture proximale (11), dans lequel dans l'espace interne (9) est disposé un bouchon de fermeture (15) de sorte que le bouchon de fermeture (15) puisse être déplacé dans l'espace interne (9) le long de l'axe central (M) et sépare une zone distale (17) de l'espace interne (9) par rapport au bouchon de fermeture (15) d'une seconde zone proximale (19) de l'espace interne (9) par rapport au bouchon de fermeture (15), le récipient pour médicaments (1) comportant une pièce de fixation de bouchon (39) qui peut être introduite au moins partiellement à travers l'ouverture proximale (11) dans la seconde zone (19) de l'espace interne (9), la pièce de fixation de bouchon (39) en état introduit dans l'espace interne (9) étant disposée plus près de l'ouverture proximale (11) que le bouchon de fermeture (15), et étant maintenue fixement, par assemblage par conjugaison de formes et/ou de forces, au moins dans une zone de maintien (49) dans l'espace interne (9), dans lequel la pièce de fixation de bouchon (39) comporte une structure de butée (51), la pièce de fixation de bouchon (39) comportant dans la zone de la structure de butée (51) au moins une extension radiale - perpendiculaire à l'axe central (M) en étant introduit - qui est plus grande qu'une extension radiale minimale de l'ouverture proximale (11) du corps de base, de sorte qu'une profondeur d'introduction de la pièce de fixation de bouchon (39) dans le récipient pour médicaments (1) est limitée par butée de la structure de butée (51) contre le récipient pour médicaments (1), **caractérisé en ce que** la pièce de fixation de bouchon (39) est conçue perméable à l'air.

2. Récipient pour médicaments (1) selon la revendication 1, **caractérisé en ce que** la pièce de fixation de bouchon (39) est maintenue fixement et/ou disposée par rapport au bouchon de fermeture (15) de telle sorte qu'un déplacement du bouchon de fermeture (15) soit limité en direction proximale par la pièce de fixation de bouchon (39), la pièce de fixation de bouchon (39) limitant le déplacement du bouchon de fermeture (15) en direction proximale par butée d'une extrémité proximale du bouchon de fermeture (15) à une extrémité distale de la pièce de fixation de bouchon (39).

3. Récipient pour médicaments (1) selon la revendication 1 ou 2, **caractérisé en ce que** la pièce de fixation de bouchon (39) collabore par conjugaison de formes et/ou de forces avec la paroi interne (47) du corps de base (7).

4. Récipient pour médicaments (1) selon l'une des revendications précédentes, **caractérisé en ce que** la pièce de fixation de bouchon (39) comporte au moins un moyen de retenue élastique (41) avec une élasticité s'exerçant de préférence radialement par rapport à l'axe central (M), le moyen de retenue élastique (41) étant conçu pour retenir par force la pièce de fixation de bouchon (39) - dans l'état introduit dans son espace interne (9) - contre le corps de base (7).

5. Récipient pour médicaments (1) selon l'une des revendications précédentes, **caractérisé en ce que** le moyen de retenue élastique (41) comporte
a) un ressort, et/ou
b) une structure élastique, et/ou
c) un élément élastique, et/ou
d) un élastomère vulcanisé, et/ou
e) une zone de matériau affaibli (43) dans laquelle un matériau voisin peut pénétrer par déformation élastique.

6. Récipient pour médicaments (1) selon l'une des revendications précédentes, **caractérisé en ce que** la pièce de fixation de bouchon (39) comporte, au moins dans une zone distale de la pièce de fixation de bouchon (39), une section ronde, en croix ou en I.

7. Récipient pour médicaments (1) selon l'une des revendications précédentes, **caractérisé en ce que** la pièce de fixation de bouchon (39) comporte dans la zone de la structure de butée (51) au moins une autre extension radiale - dans l'état introduit perpendiculaire à l'axe central (M) - plus grande qu'une extension radiale maximale de l'ouverture proximale (11) du corps de base, de sorte qu'une profondeur d'introduction de la pièce de fixation de bouchon (39) dans le récipient pour médicaments (1) soit limitée par butée de la structure de butée (51) contre le récipient pour médicaments (1).

8. Récipient pour médicaments (1) selon l'une des revendications précédentes, **caractérisé en ce qu'**à une extrémité proximale - dans un état introduit - de la pièce de fixation de bouchon (39) est constituée une zone de préhension (55), qui comporte
a) une structure de contre-dépouille (57), et/ou
b) un matériau adhérent, et/ou
c) une structure de surface rugueuse.

9. Récipient pour médicaments (1) selon l'une des revendications précédentes, **caractérisé en ce que** le récipient pour médicaments (1) comporte un indicateur de sécurité ou un tuyau rétractable qui - dans un état introduit de la pièce de fixation de bouchon (39) - dans une zone dans laquelle la pièce de fixation de bouchon (39) jouxte le corps de base (7), est disposé contre le corps de base (7) et contre la pièce de fixation de bouchon (39), de sorte que l'indicateur de sécurité puisse être modifié par un déplacement de la pièce de fixation de bouchon (39).

10. Récipient pour médicaments (1) selon l'une des revendications précédentes, **caractérisé en ce que** la pièce de fixation de bouchon (39) comporte du polypropylène, du polystyrène et/ou au moins une résine.

11. Récipient pour médicaments (1) selon l'une des revendications précédentes, **caractérisé en ce que** la pièce de fixation de bouchon (39) comporte un support d'informations lisible par une machine.

12. Récipient pour médicaments (1) selon l'une des revendications précédentes, **caractérisé en ce que** la pièce de fixation de bouchon (39) est conformée de telle sorte qu'elle ne cache - dans l'état introduit - aucune surface externe circonférentielle du récipient pour médicaments (1).

13. Utilisation d'une pièce de fixation de bouchon (39) pour sécuriser un bouchon de fermeture (15) dans un récipient pour médicaments (1) selon l'une des revendications 1 à 12, dans lequel le récipient pour médicaments (1) comporte un corps de base (7) qui entoure au moins par endroits un espace interne (9), comporte un axe central (M) et une ouverture proximale (11), dans lequel dans l'espace interne (9) est disposé un bouchon de fermeture (15) de sorte que le bouchon de fermeture (15) puisse être déplacé dans l'espace interne (9) le long de l'axe central (M) et sépare une zone distale (17) de l'espace interne (9) par rapport au bouchon de fermeture (15) d'une seconde zone proximale (19) de l'espace interne (9) par rapport au bouchon de fermeture (15), **caractérisée en ce que** la pièce de fixation de bouchon (39) est introduite à travers l'ouverture proximale (11) dans la seconde zone (19), de telle sorte qu'elle soit disposée plus près de l'ouverture proximale (11) que le bouchon de fermeture (15), la pièce de fixation de bouchon (39) étant retenue fixement par assemblage par conjugaison de formes et/ou de forces, au moins dans une zone de maintien (49) de la seconde zone proximale du récipient pour médicaments (1).

14. Utilisation d'une pièce de fixation de bouchon (39) selon la revendication 13, **caractérisée en ce que** le déplacement du bouchon de fermeture (15) est limité par la pièce de fixation de bouchon (39).
